# EUROPÄISCHE PATENTSCHRIFT

(11) **EP 1 395 361 B1**
(45) Veröffentlichungstag und Bekanntmachung des Hinweises auf die Patenterteilung: **12.07.2006**
(21) Anmeldenummer: 02719887.8
(22) Anmeldetag: 20.02.2002
(51) Int. Cl.: B01J 29/06, B01J 29/068, B01J 29/12

(54) **KATALYSATOR FÜR SÄUREKATALYSIERTE KOHLENWASSERSTOFF-UMWANDLUNGEN**
CATALYST FOR ACID-CATALYSED BY HYDROCARBON CONVERSIONS
CATALYSEUR POUR CONVERSIONS D'HYDROCARBURES A CATALYSE ACIDE

(30) Priorität: 22.05.2001 DE 10124998
(43) Veröffentlichungstag der Anmeldung: 10.03.2004
(73) Patentinhaber: SÜD-CHEMIE AG, 80333 München (DE)
(72) Erfinder: LERCHER, Johannes, A., 85521 Ottobrunn (DE); FELLER, Andreas, 80469 München (DE); GAAB, Stefan, 85748 Garching (DE)
(74) Vertreter: Westendorp, Michael Oliver
(86) Internationale Anmeldenummer: PCT/EP2002/001822
(87) Internationale Veröffentlichungsnummer: WO 2002/094436

(56) Entgegenhaltungen:
- US-A- 3 251 902
- US-A- 3 647 916
- US-A- 3 840 613
- US-A- 3 867 307
- US-A- 3 893 942
- US-A- 4 377 721
- HAAS A ET AL: "FCC catalysts containing the high-silica faujasites EMO and EMT for gas-oil cracking" MICROPOROUS AND MESOPOROUS MATERIALS, ELSEVIER SCIENCE PUBLISHING, NEW YORK, US, Bd. 28, Nr. 2, April 1999 (1999-04), Seiten 325-333, XP004223117 ISSN: 1387-1811
- OCCELLI M L ET AL: "The effects of Na ions on the properties of calcined rare-earths Y (CREY) zeolites" APPLIED CATALYSIS A: GENERAL, ELSEVIER SCIENCE, AMSTERDAM, NL, Bd. 183, Nr. 1, 5. Juli 1999 (1999-07-05), Seiten 53-59, XP004271829 ISSN: 0926-860X
- BORADE R B ET AL: "CHARACTERIZATION OF ACID SITES IN BETA AND ZSM-20 ZEOLITES" JOURNAL OF PHYSICAL CHEMISTRY, AMERICAN CHEMICAL SOCIETY, US, Bd. 96, 1992, Seiten 6729-6737, XP000852676 ISSN: 0022-3654

## Beschreibung

Die Erfindung betrifft einen Katalysator gemäß Anspruch 1.

So beschreibt die DE-B-197 45 548 einen Katalysator für die Alkylierung von C₂ bis C₅-Olefinen mit Isoparaffinen zur Erzeugung eines hochoctanhaltigen Zusatzmaterials für Kraftstoffe. Der Katalysator besteht aus einem hochporösen, ionenausgetauschten kristallinen Aluminiumsilicat (z.B. einen Zeolith Y oder X), auf das in feinverteilter Form ein Metall der Gruppe VIII des Periodensystems (z.B. Pd oder Pd/Ni) in einer Menge von 0,02 bis 0,50 Gew.-% aufgetragen ist und das folgende chemische Zusammensetzung aufweist:

REₐM_{b}Na_{c}((Al₁Siₓ)O₂₍₁₊ₓ₎).yH₂O,

wobei RE ein aus der Gruppe Lanthan und der Lanthaniden ausgewähltes Element außer Cer ist und M ein Erdalkalimetall, wie Ca ist, und wobei bedeuten:
a = 0,10 bis 0,30; b = 0,05 bis 0,25; c = 0,01 bis 0,05;
x = 1,0 bis 3,0; y = 3,0 bis 5,0.

Der Katalysator hat aufgrund seines relativ hohen Erdalkalimetallgehalts eine sehr geringe Konzentration an sauren Zentren des Brönsted-Typs.

Die US-A-3 676 368 (DE-A-2 142 270) beschreibt einen Alkali-Faujasit mit einem SiO₂/Al₂O₃-Molverhältnis von mehr als 3, der 6 bis 14 Gew.-% Ionen Seltener Erden (als Oxide) enthält, und der nach folgendem Verfahren hergestellt wurde:
a) Austausch eines Alkali-Faujasits mit einem SiO₂/Al₂O₃₋Verhältnis von etwa 3 bis 6 mit einer Lösung von Ionen Seltener Erden bei einem pH-Wert von 3,0 bis 3,5, um den Alkaligehalt auf weniger als etwa 4 Gew.-% zu vermindern;
b) Calcinieren des ausgetauschten Faujasits bei einer Temperatur von etwa 427 bis 760°C über 1 bis 3 Stunden, und
c) Austauschen des calcinierten Faujasits mit einer Lösung von Ammoniumionen, um den Alkaligehalt auf weniger als 0,5 Gew.-% herabzusetzen.

Wesentlich bei diesem Verfahren ist die Reihenfolge des Austausches des Alkalimetalls durch die Seltenen Erden und des Austausches mit Ammoniumionen. Der modifizierte Faujasit wird zur Umwandlung von Kohlenwasserstoffen verwendet.

Nach einer in der US-A-3 867 307 beschriebenen Weiterentwicklung wird der Faujasit mit einem Gemisch aus 10 bis 13 Gew.-% Ionen Seltener Erden und 0,5 bis 5 Gew.-% Ionen eines Übergangsmetalls aus der Gruppe Zink, Cadmium, Kalium, Zirkon, Mangan, Kobalt, Nickel und Kupfer ausgetauscht. Die meisten dieser Metalle bilden schwächere Brönsted-Zentren. Der modifizierte Faujasit enthält weniger als 3 Gew.-% Alkali.

Die US-A-4 377 721 beschreibt ein Verfahren zur Alkylierung von Isoparaffinen mit Olefinen in Gegenwart eines Katalysators in Form eines synthetischen kristallinen Zeoliths vom ZSM-20-Typ. Der Katalysator hat ein ungünstiges Verhältnis von sauren Zentren vom Brönsted-Typ zum Lewis-Typ.

Die US-A-5 073 665 beschreibt ein Verfahren zur Alkylierung von Olefinen mit Isoparaffinen unter Verwendung eines Katalysators in Form eines unpromotierten synthetischen Zeoliths MCM-22. Der Katalysator wurde dampfbehandelt, was zu einer Dealuminierung des Kristallgitters führt.

Ein ähnliches Verfahren betrifft die WO 97/20787, wobei als Katalysator ein Faujasit verwendet wird, dessen aktive Zentren zu mindestens 30% durch Kationen Seltener Erden ausgetauscht sind. Diese Katalysator wurde vorsätzlich dealuminiert.

Die WO 97/45383 betrifft ein Verfahren zur Alkylierung von Olefinen mit Isoparaffinen unter Verwendung eines Zeoliths mit großen Poren und einer Teilchengröße im Bereich von 20 bis 200µm. Als Katalysatoren können Faujasite und Zeolithe X und Y verwendet werden. Die Zeolithe enthalten Kationen von Seltenen Erdmetallen und nicht mehr als 1,0 Gew.-% Natrium. Nähere Angaben zum Katalysator werden nicht gemacht. Die Selektivität zur gewünschten Isooctanfraktion ist gering, d.h. es wird ein hoher Anteil an Crackprodukten erhalten, was den Dampfdruck des Kraftstoffs stark erhöht.

Die US-A-3 839 228, 4 300 015, 3 865 894 und 3 803 256 betreffen Verfahren zur Alkylierung von Olefinen mit Isoparaffinen unter Verwendung von sauren Zeolithen, die auch Ionen seltener Erden enthalten können. Die Katalysatoren haben ein ungünstiges Verhältnis zwischen den sauren Zentren vom Brönsted- und Lewis-Typ.

Die US-A-3 251 902 betrifft ein Verfahren zur Alkylierung von verzweigtkettigen Kohlenwasserstoffen unter Verwendung eines kristallinen Aluminiumsilicat-Katalysators mit Porenöffnungen von mindestens 7Å. Der Katalysator kann ein Seltene Erdmetalle enthaltender Faujasit sein. Außerdem kann er H⁺-Ionen enthalten. Der Katalysator hat ein ungünstiges Verhältnis zwischen sauren Zentren vom Brönsted- und Lewis-Typ.

Die US-A-3 647 916 betrifft ein Verfahren zum Alkylieren von verzweigtkettigen Kohlenwasserstoffen durch Umsetzen eines C₄ bis C₂₀-Isoparaffins mit einem C₂ bis C₁₂-Olefin unter Verwendung eines kristallinen Aluminosilicat-Katalysators, z.B. eines Seltenen Erden oder H⁺-Ionen enthaltenden Faujasits. Der Katalysator enthält noch einen verhältnismäßig hohen Anteil an Natrium-Ionen.

In der US 3,840,613 A wird ein Alkylierungsverfahren beschrieben, bei dem ein paraffinhaltiger Kohlenwasserstoff mit einem Olefin in Gegenwart eines kristallinen Alumosilicat-Zeolith-haltigen Katalysators in Berührung gebracht wird, der nach folgenden Stufen aktiviert wurde:
a. Basenaustausch mit einer Lösung, enthaltend Ammoniumionen;
b. Erhitzen des Zeolithprodukts von Stufe a) in Gegenwart von Wasserdampf bei Temperaturen im Bereich von 600 bis 1200°F (316 bis 650°C);
c. Basenaustausch des Zeolithprodukts von Stufe b) mit einer Lösung von Ammoniumionen, Wasserstoffionen und deren Gemische, um den Alkaligehalt auf 0.02 bis 1,0 Gew.-% herabzusetzen; und
d. Calcinieren des Zeolithproduktes von Stufe c) in Luft bei erhöhten Temperaturen.

Bei dem in Beispiel 4 beschriebenen Katalysator liegt der "Soda"-Gehalt (gemeint ist wahrscheinlich der Na₂O-Gehalt) bei 0,31 Gew.-%, was 0,23 Gew.-% Natrium entspricht. Nach den Beispielen 7 und 8 enthalten die mit NH₄ und Lanthan ausgetauschten Zeolithe noch 0,19 Gew.-% Na₂O, was 0,14 Gew.-% Natrium entspricht.

Bei der in Stufe (b) durchgeführten Dampfbehandlung wird Extragitteraluminium gebildet, welches wiederum starke Lewis-Säurenzentren bildet. Dies führt zu Katalysatoren mit niedrigem Verhältnis der Brønstedt- und Lewis-Säurezentren (B/L-Verhältnis). Wird eine Probe nach Beispiel 4 der US-A-3,840,613 hergestellt und mittels Pyridin-Adsorption charakterisiert, wird ein B/L-Verhältnis (gemessen nach Desorption des schwächer gebundenen Pyridins bei 450°C) von 0,8 gefunden. Der Anteil des bei 450°C noch an Brønsted-Säurezentren gebundenen Pyridins, bezogen auf die Menge des bei 100°C an Brønsted-Säurezentren chemisorbierten Pyridins beträgt 27%. Wird eine Probe nach Beispiel 8 der US-A-3,840,613 hergestellt und in der gleichen Weise charakterisiert, beträgt der Anteil des bei 450°C noch an Brønsted-Säurezentren gebundenen Pyridins, bezogen auf die Menge des bei 100°C an Brønsted-Säurezentren chemisorbierten Pyridins 33%. Das B/L-Verhältnis (gemessen nach Desorption des schwächer gebundenen Pyridins bei 450°C) beträgt 0,95.

In der US 3,893,942 A wird ein Alkylierungsverfahren unter Verwendung eines zeolithischen Molekularsiebs beschrieben, das eine Hydrierkomponente (Nickel, Platin, Palladium, Rhodium und Ruthenium) enthält. Der Alkalimetallgehalt des Molekularsiebes beträgt weniger als 3,5 Gew.-%, bezogen auf den entwässerten Zeolithen, das SiO₂/Al₂O₃-Molver-hältnis beträgt mindestens 2,0. Wird analog zur US 3,893,942 A eine Probe des zeolithischen Molekularsiebs und auf seine Brønstedt- und Lewis-Säurezentren untersucht, beträgt, bezogen auf die Masse des bei 100°C an die Brønsted-Säurezentren gebundenen Pyridins, der Anteil des bei 450°C noch an Brønsted-Säurezentren gebundenen Pyridins 0,9%. Die Rest-Natriumkonzentration beträgt 0,3%.

In der US 3,867,307 A wird ein Faujasit beschrieben, dessen Alkaliionen teilweise durch die Ionen seltener Erden und teilweise durch zweiwertige Ionen (Zink, Cadmium, Thallium, Zirkon, Mangan, Kobalt, Nickel und Kupfer) ausgetauscht sind. Die zweiwertigen Ionen bilden zwar auch Säurezentren vom Brønsted-Typ. Wird jedoch eine Probe nach der Arbeitsweise von Beispiel 1 der US 3,867,307 A hergestellt und durch Pyridin-Adsorption charakterisiert, besitzt eine so hergestellte Probe ein B/L Verhältnis (gemessen nach Desorption des schwächer gebundenen Pyridins bei 450°C) von 0,07. Der Anteil des bei 450°C noch an Brønsted-Säurezentren gebundenen Pyridins von der Menge des bei 100°C Brønsted-Säurezentren gebundenen Pyridins beträgt 2,1%.

In der US 3,251,902 A wird ein Verfahren zum Alkylieren von verzweigtkettigen Kohlenwasserstoffen (Isobutan bzw. Isopentan) mit einem C₂ bis C₅-Olefin in Gegenwart eines kristallinen Alumosilicats bestimmter Porengröße beschrieben. Die Alkaliionen des Alumosilicats werden durch die Ionen von seltenen Erden ausgetauscht und das ausgetauschte Material wird einer Dampfbehandlung unterzogen. Der Ionenaustausch geht jedoch nicht so weit, dass die Konzentration der Alkaliionen auf weniger als 0,2 Gew.-% vermindert wird. Der Gehalt an restlichen Natriumionen kann bis zu 3,5 Gew.-% betragen.

In der US 3,647,916 A wird ein Verfahren zur Alkylierung von verzweigtkettigen Isoparaffinen mit Olefinen unter Verwendung eines Katalysators aus kristallinem Alumosilicat mit Porenöffnungen von mindestens 7 Å beschrieben. Die in den Beispielen aufgeführten Alkylierungskatalysatoren sind Faujasite, die durch Austausch mit den Ionen von seltenen Erden und Ammonium mit nur einer Calcinierung hergestellt werden.

In den Beispielen 1 bis 3 der US-A-3,647,916 werden ein RE-X, ein RE-H-Y und ein H-Y Zeolith beschrieben, die als AlkylierungsKatalysatoren eingesetzt werden. Die Probe RE-X (Beispiel 1) wurde soweit mit Seltenerdionen ausgetauscht, dass 97% des Natriums ersetzt sind. Die verbleibenden 3% entsprechen einer Rest-Natriumkonzentration von 0,35 bis 0,4 Gew.-% (bei einer typischen Ausgangskonzentration von etwa 12 Gew.-% eines Na-X). Werden für die Probe RE-H-Y aus Beispiel 2 der US-A-3,647,916 die Brønstedt- und Lewis-Säurezentren durch Adsorption von Pyridin bestimmt, wird ein B/L Verhältnis < 1,4 gefunden. Wird die Probe H-Y aus Beispiel 3 aus der US-A-3,647,916 hergestellt und durch Pyridin-Adsorption untersucht, besitzt eine solcherart hergestellt Probe ein B/L-Verhältnis (gemessen nach Desorption des schwächer gebundenen Pyridins bei 450°C) von 0,01. Der Anteil des bei 450°C noch an Brønsted-Säurezentren gebundenen Pyridins, bezogen auf die Menge des bei 100°C Brønsted-Säurezentren gebundenen Pyridins beträgt 0,8%.

In der US 4,377,721 A wird ein Verfahren zur Alkylierung von Isoparaffinen mit Olefinen in Gegenwart eines Katalysators beschrieben, welcher einen synthetischen kristallinen Zeolith enthält, der ein bestimmtes Muster von Röntgenbeugungslinien zeigt. Die Alkylierungskatalysatoren basieren auf ZSM-5-20-Zeolithen, welche mit Faujasiten verwandt sind. Die Probe REHY aus Beispiel 1 der US 4,377,721 A weist ein B/L-Verhältnis von etwa 1,3 auf.

Haas et al., Microp. Mesop. Mater. 28, 325-333 (1999) beschreiben die Verwendung von EMT und EMO, d.h. faujasitähnlichen Zeolithen als Crackkatalysatoren. Diese werden in einem ersten Austauschschritt mit den Ionen seltener Erden bzw. mit Ammoniumionen auf einem Restnatriumgehalt von 4 Gew.-% gebracht. Im zweiten Austauschschritt werden alle Proben mit Ammoniumionen auf einen Restnatriumgehalt von weniger als 1 Gew.-% gebracht.

Ocelli et al., Appl. Catal. A 183, 53-59 (1999) beschreiben Faujasite (Zeolith Y), welche mit Ionen von seltener Erden ausgetauscht wurden. Diese Faujasite können als Crackkatalysatoren Verwendung finden. Weiterhin werden diese Faujasite nach einem oder mehreren Austauschschritten mit Ammoniumionen calciniert.

Borade et al., J. Phys. Chem. 96, 6729-6737 (1992), untersuchten verschiedene Zeolith-Typen, die durch Austausch mit Ammoniumionen in die saure Form gebracht werden, auf ihre Säureeigenschaften. Eine der Messmethoden umfasst die Adsorption von Pyridin, gemessen in einem IR-Gerät. Allerdings wird das Verhältnis der Säurezentren von Brønsted- und Lewis-Typ bei 200°C gemessen, wobei wesentlich höhere Werte zu erwarten sind, als bei einer Messung bei 450°C

Der Erfindung liegt die Aufgabe zugrunde, einen Katalysator für säurekatalysierte Kohlenwasserstoff-Umwandlungen, insbesondere für die Alkylierung von Olefinen mit Isoparaffinen, auf der Basis von kristallinen Zeolithen bereitzustellen, der bei einer hohen Aktivität eine ausgezeichnete Selektivität hat. Insbesondere soll einerseits die Bildung von Crackprodukten, andererseits die Bildung von polymeren Abscheidungen auf und in den Poren des Katalysators minimiert und die Lebensdauer des Katalysators maximiert werden.

Gegenstand der Erfindung ist somit ein Katalysator für säurekatalysierte Kohlenwasserstoff-Umwandlungen, insbesondere für die Alkylierung von Olefinen mit Isoparaffinen, enthaltend einen kristallinen Faujasit vom Typ X, Y oder LSX mit einen SiO₂/Al₂O₃-Molverhältnis von < 10, dessen Alkali-Kationen mindestens teilweise durch drei- oder mehrwertige Kationen ausgetauscht sind, wobei die Restkonzentration an Alkali-Kationen weniger als etwa 0,2 Gew.-% beträgt, wobei die Konzentration der Brönsted-Zentren, bestimmt als Funktion des an der Oberfläche chemisorbierten Pyridins, 0,1 bis 4 mmol/g Katalysator beträgt und das Verhältnis zwischen der Konzentration der Säurezentren vom Brönsted-Typ (B) und vom Lewis-Typ (L), ausgedrückt als Flächenverhältnis der Absorptionsbanden bei 1540 ± 5cm⁻¹ (B) und 1450 ± 5cm⁻¹ (L) nach dem Erhitzen auf eine Temperatur von 450°C 1,4 oder höher beträgt, wobei der Anteil des bei einer Temperatur von 450°C noch nicht von der Katalysatoroberfläche desorbierten, an Brönsted-Säurezentren gebundenen Pyridins 30 bis 60% des bei 100°C an Brönsted-Säurezentren chemisorbierten Pyridins beträgt.

Für solche zeolithische Alkylierungskatalysatoren wurde überraschend gefunden, dass sie eine möglichst hohe Konzentration an Brönsted- Säurezentren einer bestimmten Stärke und eine geringe Konzentration an starken Lewis-Säurezentren aufweisen. Die letzteren Zentren entstehen gewöhnlich durch Aluminium-Kationen, die während des Modifikationsprozesses, insbesondere während der Calcinierungsschritte, aus dem Kristallgitter herausgelöst werden. Je größer das Ausmaß dieses Prozesses, um so niedriger wird die Konzentration der Brönsted-Säurezentren und um so höher die der Lewis-Säurezentren. Lewis-Säurezentren sind katalytisch inaktiv, binden jedoch Olefine, wodurch die Oligomerisierung beschleunigt wird. Die an die Brönsted-Säurezentren gebundenen Carbeniumionen desorbieren mit Hilfe von Isobutan, über den Schritt des Hydridtransfers, als Alkane.

Eine hohe Konzentration an Brönsted-Säurezentren führt einerseits zu hoher Aktivität des Katalysators, da eine große Anzahl von Carbeniumionen im Reaktionsvolumen vorliegt. Andererseits wurde überraschend beobachtet, dass die Stabilität der Carbeniumionen mit steigender Konzentration an Aluminium geringer und ihre Ablösung durch Hydridtransfer von Isobutan deutlich erhöht wird. Dies führt zu einer Unterdrückung von Spaltreaktionen und Oligomerisierung und bewirkt eine höhere Lebensdauer sowie Selektivität des Katalysators.

Die Bestimmung der adsorbierten Menge an Pyridin und des Brönsted/Lewis-Säurezentrenverhältnisses geschieht in einem Infrarot-Spektrometer. Dazu wird die Katalysatorprobe bindemittelfrei zu einer Tablette gepresst und in eine Messzelle gebracht, die anschließend evakuiert wird. Die Probe wird etwa 1 Stunde bei 450°C aktiviert. Danach wird Pyridin bei einer Temperatur von etwa 100°C als Gas mit einem Druck von etwa 10⁻² mbar eingeführt. Die Sättigung ist erreicht, wenn die Hydroxylbanden bei 3640 ± 10 und 3600 ± 10 cm⁻¹ verschwunden sind. Danach wird die Probe bei 10⁻⁶ mbar evakuiert, um schwächer gebundenes Pyridin zu entfernen. Nach temperaturprogrammiertem Aufheizen der Probe auf 450°C wird das Flächenverhältnis der Absorptionsbanden des Pyridins bei 1540 ± 5 cm⁻¹ (B) und 1450 ± 5 cm⁻¹ (L) vermessen.

Bevorzugte Ausführungsformen sind in den Unteransprüchen angegeben.

Vorzugsweise sind die Alkaliionen des kristallinen Faujasits zumindest teilweise durch H⁺-Ionen ersetzt.

Vorzugsweise beträgt das Verhältnis zwischen der Konzentration der Zentren vom Brönsted-Typ (B) und vom Lewis-Typ (L) 1,5 bis 6, insbesondere 1,5 bis 5.

Das SiO₂/Al₂O₃-Molverhältnis ist vorzugsweise < 7, insbesondere 2 bis 6.

Die mehrwertigen Kationen sind vorzugsweise Kationen der Seltenen Erdmetalle.

Die H⁺-Form des Zeoliths ist vorzugsweise durch Austausch der Alkali-Kationen durch NH₄⁺-Ionen und anschließendes Calcinieren erhältlich.

Die Konzentration der Brönsted-Säurezentren beträgt vorzugsweise 0,2 bis 4, insbesondere 0,5 bis 2mmol/g Katalysator.

Der Katalysator kann zusätzlich mindestens ein Metall der 8. Nebengruppe, insbesondere ein Metall der Platingruppe, in einer Menge von 0,01 bis 0,5 Gew.-% enthalten.

Durch den Zusatz eines Metalls der 8. Nebengruppe kann der Katalysator leichter regeneriert werden, wodurch seine Gesamtlebensdauer erhöht wird. Aus der US-A-3 893 942 ist der Zusatz von Edelmetallen, wie Platin, bekannt. Es ist dort angegeben, dass ein teilweise deaktivierter zeolithischer Katalysator, welcher mit Platin ausgetauscht wurde, durch Behandlung mit Wasserstoff regeneriert werden kann. Bei der Regenerierung der Katalysatoren werden die in den Poren sitzenden polymeren Produkte hydrierend gespalten und aus dem katalytischen Material entfernt. Auf die Alkylierung haben die Metalle der 8. Nebengruppe keinen Einfluss.

Die Regenerierung kann im Prinzip auch durch Behandlung des mit den Polymeren beladenen Katalysators mit Sauerstoff oder einem sauerstoffhaltigen Gas erfolgen. Diese Reaktion ist aber stark exotherm, weshalb sie weniger bevorzugt wird.

Gegenstand der Erfindung ist ferner die Verwendung des erfindungsgemäßen Katalysators für säurekatalysierte Kohlenwasserstoff-Umwandlungen, insbesondere für die Alkylierung von Olefinen mit Isoparaffinen, z.B. von C₂₋₆-Olefinen mit C₄-C₆ Isoparaffinen. Die Umsetzung wird bei Temperaturen von etwa 0 bis 200°C, insbesondere von etwa 50 bis 120°C, bevorzugt von etwa 60 bis 100°C durchgeführt, und zwar vorzugsweise in der flüssigen Phase, wobei mindestens eines der Edukte bei der Reaktionstemperatur flüssig ist. Zu diesem Zweck führt man die Umsetzung vorzugsweise bei etwa 4 bis 40 bar durch.

Es ist aber auch eine Umsetzung in der Gasphase denkbar.

Die Erfindung ist durch die nachstehenden Beispiele in nicht einschränkender Weise erläutert.

### Beispiel 1

50g eines kommerziellen Zeoliths X mit einem SiO₂/Al₂O₃-Molverhältnis von 2,6 und einem Natriumgehalt von 14 Gew.-% wurden in 300ml einer 0,2-molaren Lanthannitratlösung aufgeschlämmt und unter Rühren 2 Std. bei 80°C erhitzt. Die Lösung wurde abgefiltert, und der Filterkuchen wurde ohne Waschen nochmals mit 300ml 0,2 molarer Lanthannitratlösung behandelt. Der nach dem Abfiltrieren und Waschen erhaltene Filterkuchen hatte einen Natriumgehalt von 2 Gew.-%. Der Filterkuchen wurde über Nacht bei 100°C getrocknet, danach gemahlen und in einem Röhrenofen im Luftstrom calciniert, wobei die Temperatur über einen Zeitraum von 8 Std. von 120°C auf 200°C erhöht wurde, worauf mit 3°C/min auf 450°C hochgeheizt wurde. Diese Temperatur wurde noch 1 Std. konstant gehalten.

Dann wurde das calcinierte Produkt, wie oben beschrieben, 4 weiteren Lanthanaustauschschritten unterzogen. Der nach dem Waschen und Abfiltrieren erhaltene Filterkuchen wurde wie vorstehend angegeben calciniert. Das Produkt hatte einen Natriumgehalt von 0.05 Gew.-%.

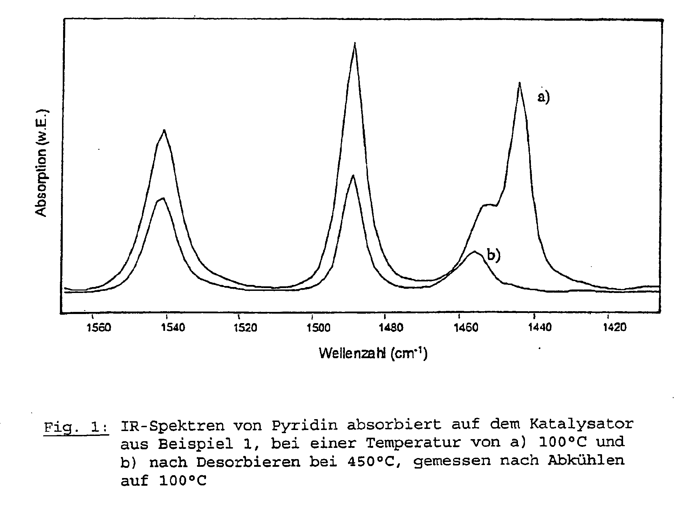

Die erhaltene Probe wurde wie oben beschrieben IR-spektroskopisch untersucht. Das nach dem Aufheizen auf 450°C gemessene Verhältnis von Brönsted- zu Lewis-Säurezentren betrug 2,9. Das IR-Spektrum bei 100°C und nach dem Aufheizen auf 450°C ist in Fig. 1 dargestellt. Nach dem Erhitzen auf 450°C betrug der Anteil des Brönsted-gebundenen Pyridins 55% der bei 100°C gebundenen Gesamtmenge an Pyridin.

### Beispiel 2

Die Arbeitsweise von Beispiel 1 wurde mit der Abweichung wiederholt, dass die Probe vor dem zweiten Calcinieren zusätzlich noch mit 400ml einer 0,05 molaren Ammoniumnitratlösung ebenfalls 2 Std. bei 80°C behandelt wurde. Der nach dem Waschen und Abfiltrieren erhaltene Filterkuchen wurde wie in Beispiel 1 angegeben calciniert. Während der Calcinierung wurde die Menge an Ammoniak desorbiert, die einer H⁺⁻Konzentration von etwa 0,01 mmol/g Katalysator entspricht. Die Gesamtkonzentration an sauren Brönsted-Zentren wurde zu 0,57 mmol/g bestimmt. Der Katalysator hatte einen Restnatriumgehalt von 0,04 Gew.-%. Das nach dem Aufheizen auf 450°C gemessene Verhältnis von Brönsted- zu Lewis-Säurezentren betrug 2,4. Der Anteil des Brönsted-gebundenen Pyridins bei 450°C betrug 37% des bei 100°C gebundenen Pyridins.

### Beispiel 3

Die Arbeitsweise von Beispiel 1 wurde mit der Abweichung wiederholt, dass das Produkt vor dem letzten Calcinierungsschritt mit 500ml destilliertem Wasser aufgeschlämmt wurde und über einen Zeitraum von 4 Std. eine Lösung mit 0,2g Tetraminpalladium(II)Chlorid unter Rühren zugetropft wurde. Nach weiteren 24 Std. wurde die Probe gewaschen und abfiltriert. Der Filterkuchen wurde im Luftstrom wie oben beschrieben calciniert, und anschließend bei 300°C für 4 Std. im Wasserstoffstrom behandelt, wobei das Edelmetall reduziert wurde. Der Edelmetallgehalt der Probe betrug nach dem Calcinieren etwa 0,2 Gew.-%.

### Beispiel 4

An einer Serie von Proben wurde die Arbeitsweise von Beispiel 1 mit den Abweichungen wiederholt, wobei die Reihenfolge des Austauschs, die Konzentrationen der Austauschlösungen und die Zwischencalcinierung variiert wurden. Die Präparationsschritte, der Gehalt an Natrium und das B/L-Verhältnis bei 450°C sind in Tabelle 1 aufgeführt.

**Tabelle 1**

| Probe | Modifikationsweise | Na-Gehalt (Gew.-%) | B/L 450°C |
|---|---|---|---|
| 1 | 1. Stufe: La-Austausch; 2. Stufe: NH₄-Austausch | 0,09 | 1,3 |
| 2 | 1. Stufe: La-Austausch; 2. Stufe: NH₄ + La -Austausch | 0,002 | 2,0 |
| 4 | 1. Stufe: La-Austausch; 2. Stufe: NH₄ + La -Austausch | 0,002 | 1,2 |
| 5 | 1. Stufe: NH₄ + La-Austausch; keine 2. Stufe: | 0.19 | 1,3 |
| 6 | 1. Stufe: La-Austausch; 2. Stufe: NH₄ + La -Austausch | 0,03 | 1,6 |
| 7 | 1. Stufe: La-Austausch; 2. Stufe: NH₄ + La -Austausch | 0,10 | 1,6 |
| 8 | 1. Stufe: La-Austausch; 2. Stufe: NH₄ + La -Austausch | 0,05 | 2,5 |
| 9 | 1. Stufe: La-Austausch; 2. Stufe: NH₄ +.La -Austausch | 0,04 | 2,8 |
| 10 | Beispiel 1 | 0,05 | 2,9 |
| 11 | Beispiel 2 | 0,04 | 2,4 |

### Anwendungsbeispiel 1

4 bis 5g des Katalysators von Beispiel 1 wurden in einen 50ml Rührkesselreaktor eingebracht und bei 180°C mit Stickstoff über Nacht aktiviert. Nach dem Abkühlen auf die Reaktionstemperatur von 75°C wurde der Reaktor mit Isobutan unter einem Druck von 32 bar gefüllt. Das Isobutan war unter diesen Bedingungen flüssig. Nach Anfahren des Rührers auf 1800 U/min wurde eine Mischung von Isobutan und 2-Buten im Molverhältnis 6,7 : 1 durch den Reaktor geleitet. Die Raumgeschwindigkeit, bezogen auf Buten, betrug 0.2g Buten pro Gramm Katalysator und Stunde.

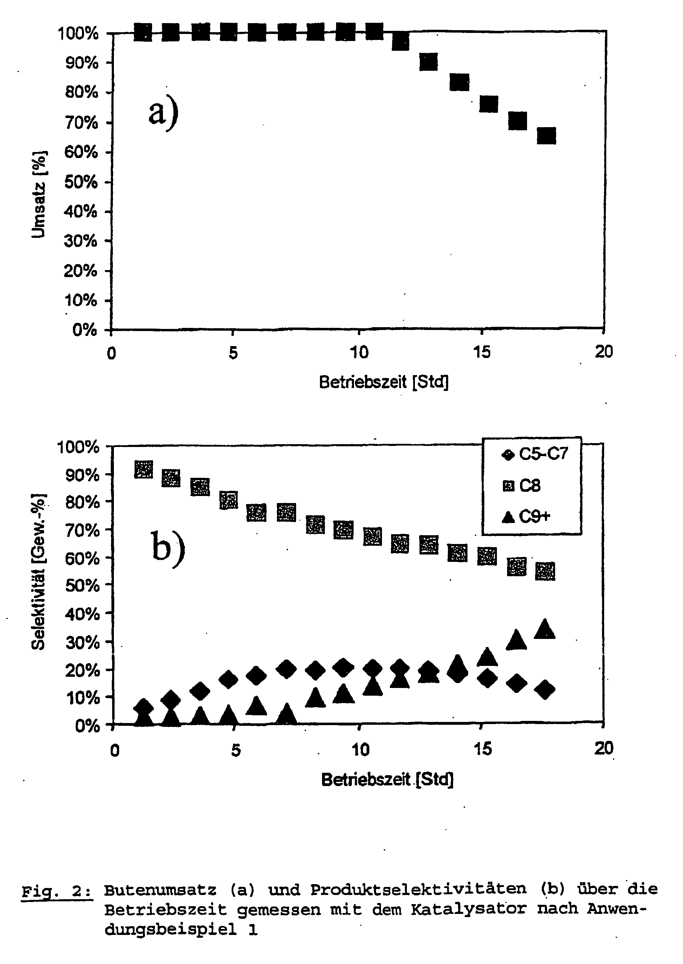

Das Produktgemisch wurde über Kopf abgezogen und durch einen Partikelfilter vom Katalysator getrennt. Der Produktstrom wurde über einen Druckregler entspannt und durch auf etwa 150 bis 180°C beheizte Leitungen durch ein 6-Wege-Ventil geleitet, welches alle 70 min eine Probe zur Analyse in einen Gaschromatographen einschleuste. Aus den Chromatogrammen wurden Selektivitäten und Umsätze berechnet. Wie in Fig. 2 dargestellt ist, betrug der Butenumsatz über einen Zeitraum von 11 Std. 100%, um dann über weitere 6 Std. auf etwa 60% abzusinken. Die Selektivität, bezogen auf das Gesamtprodukt betrug zu Beginn 92 Gew.-% Isooctan, 5 Gew.-% C₅- bis C₇-Paraffine und 3 Gew.-% C₉₊-Paraffine.

Nach 11 Std. waren die Selektivitäten, bezogen auf das Gesamtprodukt, wie folgt: 69 Gew.-% Isooctan, 17 Gew.-% C₅ bis C₇-Paraffine, 14 Gew.-% C₉₊-Paraffine. Olefinische Kohlenwasserstoffe wurden nicht detektiert.

### Anwendungsbeispiel 2

Der Katalysator nach Beispiel 2 wurde unter den gleichen Bedingungen wie nach dem Anwendungsbeispiel 1 auf die katalytische Aktivität und Selektivität untersucht. Der Butenumsatz betrug über einen Zeitraum von 12 Std. 100% und fiel dann ab. Die Selektivität, bezogen auf das Gesamtprodukt, betrug zu Beginn 93 Gew.-% Isooctan, 5 Gew.-% C₅ bis C₇-Paraffine und 2 Gew.-% C₉₊-Paraffine. Nach 12 Std. waren die Selektivitäten, bezogen auf das Gesamtprodukt, wie folgt: 68 Gew.-% Isooctan, 18 Gew.-% C₅ bis C₇-Paraffine und 14 Gew.-% C₉₊-Paraffine

### Anwendungsbeispiel 3

Der palladiumhaltige Katalysator nach Beispiel 3 wurde ebenfalls unter den gleichen Bedingungen wie nach dem Anwendungsbeispiel 1 getestet. Auch hier fiel der Butenumsatz nach 11 Std. Betriebszeit unter 100%. Die Selektivität, bezogen auf das Gesamtprodukt, betrug zu Beginn 92 Gew.-% Isooctan, 5 Gew.-% C₅ bis C₇-Paraffine und 3 Gew.-% C₉₊-Paraffine. Nach 11 Std. waren die Selektivitäten, bezogen auf das Gesamtprodukt, wie folgt: 69 Gew.-% Isooctan, 17 Gew.-% C₅ bis C₇-Paraffine und 14 Gew.-% C₉₊-Paraffine.

### Anwendungsbeispiel 4

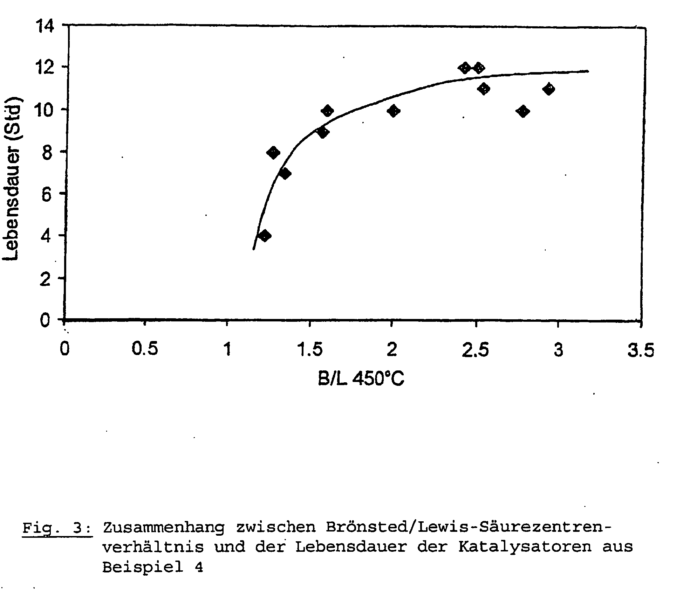

Die Katalysatoren von Beispiel 4 wurden alle unter denselben Bedingungen wie in Anwendungsbeispiel 1 getestet. Die Proben zeigten unterschiedliche Lebensdauern, definiert als den Zeitraum von 100% Butenumsatz, von 4 bis 12 Std. In Fig. 3 ist dargestellt, wie die Lebensdauer des Katalysators von dem Brönsted/Lewis-Säurezentrenverhältnis gemessen bei 450°C abhängt. Die Lebensdauer steigt mit dem B/L-Verhältnis bis zu einem Wert von 1,5 an und bleibt dann weitgehend konstant.

## Patentansprüche

1. Katalysator für säurekatalysierte Kohlenwasserstoff-Umwandlungen, insbesondere für die Alkylierung von Olefinen mit Isoparaffinen, enthaltend einen kristallinen Faujasit vom Typ X, Y oder LSX, mit einem SiO₂/Al₂O₃-Molverhältnis von < 10, dessen Alkali-Kationen mindestens teilweise durch drei- oder mehrwertige Kationen ausgetauscht sind, wobei die Restkonzentration an Alkali-Kationen weniger als 0,2 Gew.-% beträgt, wobei die Konzentration der Brönsted-Säurezentren, bestimmt als Funktion des an der Oberfläche chemisorbierten Pyridins, 0,1 bis 4mmol/g Katalysator beträgt und das Verhältnis zwischen der Konzentration der Säurezentren vom Brönsted-Typ (B) und vom Lewis-Typ (L), ausgedrückt als Flächenverhältnis der Absorptionsbanden bei 1540 ± 5cm⁻¹ (B) und 1450 ± 5cm⁻¹ (L) nach dem Erhitzen auf eine Temperatur von 450°C, 1,4 oder höher beträgt, wobei der Anteil des bei einer Temperatur von 450°C noch nicht von der Katalysatoroberfläche desorbierten, an Brönsted-Säurezentren gebundenen Pyridins 30 bis 60% des bei 100°C an Brönsted-Säurezentren chemisorbierten Pyridins beträgt.

2. Katalysator nach Anspruch 1 **dadurch gekennzeichnet, dass** die Alkaliionen des kristallinen Faujasits zumindest teilweise durch H⁺-Ionen ersetzt sind.

3. Katalysator nach Anspruch 1 oder 2 **dadurch gekennzeichnet, dass** das Verhältnis zwischen der Konzentration der Säurezentren vom Brönsted-Typ (B) und vom Lewis-Typ (L) 1,5 bis 6, vorzugsweise 1,5 bis 5, beträgt.

4. Katalysator nach einem der Ansprüche 1 bis 3 **dadurch gekennzeichnet, dass** das SiO₂/Al₂O₃-Molverhältnis < 7, vorzugsweise 2 bis 6, beträgt.

5. Katalysator nach einem der Ansprüche 1 bis 4, **dadurch gekennzeichnet, dass** die mehrwertigen Kationen Kationen der Seltenen Erdmetalle darstellen.

6. Katalysator nach einem der Ansprüche 1 bis 5, **dadurch gekennzeichnet, dass** die Konzentration der Säurezentren 0,2 bis 4, vorzugsweise 0,5 bis 2 mmol/g Katalysator beträgt.

7. Katalysator nach einem der Ansprüche 1 bis 6, **dadurch gekennzeichnet, dass** er zusätzlich mindestens ein Metall der 8. Nebengruppe, insbesondere ein Metall der Platingruppe, in einer Menge von 0,01 bis 0,5 Gew.-% enthält.

8. Verwendung des Katalysators nach einem der Ansprüche 1 bis 7, für säurekatalysierte Kohlenwasserstoff-Umwandlungen, insbesondere für die Alkylierung von Olefinen mit Isoparaffinen.

9. Verwendung nach Anspruch 8, **dadurch gekennzeichnet, dass** man die Umsetzung bei Temperaturen von 0 bis 200°C, vorzugsweise von 50 bis 120°C durchführt.

10. Verwendung nach Anspruch 8, **dadurch gekennzeichnet, dass** man die Umsetzung in der flüssigen Phase durchführt, wobei mindestens eines der Edukte bei der Reaktionstemperatur flüssig ist.

11. Verwendung nach einem der Ansprüche 8 bis 10, **dadurch gekennzeichnet, dass** man die Umsetzung bei 4 bis 40 bar durchführt.

## Claims

1. A catalyst for acid-catalysed hydrocarbon conversions, in particular for the alkylation of olefins with isoparaffins, containing a crystalline faujasite of the X,Y or LSX type, with an SiO₂/Al₂O₃ molar ratio of < 10, the alkali cations of which are at least party replaced with trivalent or polyvalent cations, wherein the residual concentration of alkali cations amounts to less than 0.2 % by weight, wherein the concentration of the Brönsted acid sites, determined as a function of the pyridine chemisorbed on the surface, amounts to 0.1 to 4 mmol/g of catalyst, and the ratio between the concentration of the acid sites of the Brönsted type (B) and of the Lewis type (L), expressed as the surface ratio of the absorption bands at 1540 ± 5 cm⁻¹ (B) and 1450 ± 5 cm⁻¹ (L) after heating to a temperature of 450°C, amounts to 1.4 or higher, wherein the proportion of the pyridine not yet desorbed from the catalyst surface at a temperature of 450°C, bound to Brönsted acid sites, amounts to 30 to 60%, of the pyridine chemisorbed at Brönsted acid sites at 100°C.

2. A catalyst according to Claim 1, **characterised in that** the alkali ions of the crystalline faujasite is replaced at least partly with H⁺ ions.

3. A catalyst according to Claim 1 or 2,
**characterised in that** the ratio between the concentration of the acid sites of the Brönsted type (B) and of the Lewis type (L) amounts to 1.5 to 6, preferably 1.5 to 5.

4. A catalyst according to any one of the Claims 1 to 3, **characterised in that** the SiO₂/Al₂O₃ molar ratio amounts to < 7, preferably 2 to 6.

5. A catalyst according to any one of the Claims 1 to 4, **characterised in that** the polyvalent cations are cations of the rare earth metals.

6. A catalyst according to any one of the Claims 1 to 5, **characterised in that** the concentration of the acid sites amounts to 0.2 to 4, preferably 0.5 to 2 mmol/g of catalyst.

7. A catalyst according to any one of the Claims 1 to 6, **characterised in that** additionally it contains at least one metal of the subgroup 8, in particular a metal of the platinum group, in an amount of from 0.01 to 0.5% by weight.

8. Use of the catalyst according to any one of the Claims 1 to 7 for acid-catalysed hydrocarbon conversions, in particular for the alkylation of olefins with isoparaffins.

9. Use according to Claim 8, **characterised in that** the conversion is carried out at temperatures of 0 to 200°C, preferably from 50 to 120°C.

10. Use according to Claim 8, **characterised in that** the conversion is carried out in the liquid phase, wherein at least one of the educts is liquid at the reaction temperature.

11. Use according to any one of Claims 8 to 10, **characterised in that** the conversion is carried out at 4 to 40 bar.

## Revendications

1. Catalyseur pour des conversions d'hydrocarbures par catalyse acide, en particulier pour l'alkylation des oléfines avec des isoparaffines, contenant une faujasite cristalline du type X, Y ou LSX, avec un rapport molaire SiO₂/Al₂O₃ de < 10, dont les cations alcalins sont remplacés au moins partiellement par des cations trivalents ou polyvalents, dans lequel la concentration résiduelle en cations alcalins atteint moins de 0,2 % en poids, la concentration en centres acides de Brönsted, déterminée en fonction de la pyridine chimisorbée à la surface, atteint 0,1 à 4 mmol/g de catalyseur et le rapport entre la concentration en centres acides de type Brönsted (B) et de type Lewis (L), exprimé sous forme du rapport surfacique des bandes d'absorption à 1540 ± 5 cm⁻¹ (B) et 1450 ± 5 cm⁻¹ (L) après chauffage à une température de 450°C, atteint 1,4 ou plus, la proportion de la pyridine liée aux centres acides de Brönsted, pas encore désorbée par la surface du catalyseur à une température de 450°C, atteignant 30 à 60 % de la pyridine chimisorbée sur les centres acides de Brönsted à 100°C.

2. Catalyseur selon la revendication 1, **caractérisé en ce que** les ions alcalins de la faujasite cristalline sont remplacés au moins partiellement par des ions H⁺.

3. Catalyseur selon la revendication 1 ou 2, **caractérisé en ce que** le rapport entre la concentration des centres acides de type Brönsted (B) et de type Lewis (L) va de 1,5 à 6, de préférence de 1,5 à 5.

4. Catalyseur selon l'une des revendications 1 à 3, **caractérisé en ce que** le rapport molaire SiO₂/Al₂O₃ est < 7, de préférence de 2 à 6.

5. Catalyseur selon l'une des revendications 1 à 4, **caractérisé en ce que** les cations polyvalents représentent des cations de métaux de terres rares.

6. Catalyseur selon l'une des revendications 1 à 5, **caractérisé en ce que** la concentration en centres acides atteint 0,2 à 4, de préférence 0,5 à 2 mmol/g de catalyseur.

7. Catalyseur selon l'une quelconque des revendications 1 à 6, **caractérisé en ce qu'**il contient en plus au moins un métal du 8^{ème} groupe secondaire, en particulier un métal du groupe du platine, en une quantité de 0,01 à 0,5 % en poids.

8. Utilisation du catalyseur selon l'une des revendications 1 à 7, pour des conversions d'hydrocarbures par catalyse acide, en particulier pour l'alkylation des oléfines avec des isoparaffines.

9. Utilisation selon la revendication 8, **caractérisée en ce que** la conversion est réalisée à des températures de 0 à 200°C, de préférence de 50 à 120°C.

10. Utilisation selon la revendication 8, **caractérisée en ce que** la conversion est réalisée en phase liquide, où au moins un des réactifs est liquide à température réactionnelle.

11. Utilisation selon l'une des revendications 8 à 10, **caractérisée en ce que** l'on réalise la conversion à une pression de 4 à 40 bars.
